# EUROPEAN PATENT APPLICATION

(11) **EP 1 826 561 A1**
(43) Date of publication of application: **29.08.2007**
(21) Application number: 05774955.8
(22) Date of filing: 19.08.2005
(51) Int. Cl.: G01N 33/53, C12M 1/00, C12N 15/09, C12Q 1/02, G01N 33/543, C12Q 1/68

(54) **A SUBSTRATE, A METHOD FOR QUANTIFICATION OF A TARGET SUBSTANCE**

(30) Priority: 13.12.2004 JP 2004360631
(71) Applicant: National University Corporation Okayama University, Okayama-shi, Okayama 700-8530 (JP)
(72) Inventor: KOSAKA, Jun, GRADUATE SCHOOL OF MEDICINE DENTISTRY, Okayama-shi, Okayama 7008558 (JP); SASAKI, Junzo, GRAD. SCHOOL OF MEDICINE DENTISTRY, Okayama-shi, Okayama 7008558 (JP)
(74) Representative: Wibbelmann, Jobst
(86) International application number: PCT/JP2005/015581
(87) International publication number: WO 2006/064591

(57) **Abstract**

The object of this invention is to provide a substrate that enables quantification of target substances as absolute values at a single cell level, and a method for such quantification. The substrate according to this invention is **characterized in that** it comprises a concentration gradient spotting portion comprising fragments having binding property with probe and having plural concentration gradients, and a specimen setting portion for setting specimen containing target substance having binding property with said probe.

## Description

### BACKGROUND OF THE INVENTION

### 1. Field of the Invention

The present invention relates to a substrate, and to a method for quantification of a target substance such as transcriptional products of a gene, particularly to a substrate that is available for quantification of a target substance utilizing affinity interaction and to such method for quantification.

### 2. Related Art

In general, northern blot, slot and dot blots, RNase protection method, RT-PCR can be listed as conventional methods for analysis of gene expression. Moreover, immunoscreening method is also adopted as a method for identification using certain affinity interaction.

Moreover, on histological or cytologic specimens, in situ hybridization method (referred to ISH) is a technique useful for investigating on RNA expression or for recognition on the roles of specific genes at normal or disease conditions.

For such example, in situ hybridization method for quantifying intracellular mRNA can be cited (PCT Publication 2001-509662). This method is characterized in quantifying the amount of target nucleic acid sequence in morphologically intact cell, comprising the steps of; A. culturing specimens of not lower than two cells which are physically distinguishable on at least one substrate, B. contacting said cell with a fixative that preserves and retains nucleic acid in the inner space of the cell, C. thereby permeating labeled nucleic acid probes into the morphologically intact cell, and exposing the fixed cell to the labeled nucleic acid probes under the condition that hybridizes with target nucleic acid sequence, and D. detecting the amount of the probes hybridized to the target nucleic acid sequence.
Patent literature 1 : PCT Publication 2001-509662

### DISCLOSURE OF THE INVENTION

However, above-mentioned techniques such as northern blot, slot and dot blots, RNase protection method, RT-PCR have delicate protocols for investigation on alteration of mRNA expression levels, therefore, they have deficiencies in lack of quantitative determination, low sensitivity and inappropriate reproducibility. Furthermore, in each of these approaches, the manipulation of isolating target mRNA pool is required prior to analysis. These factors have problems of restricting the amount of specimen to be treated strictly, increasing the time for analysis and reducing reliability.

As to in situ hybridization method described above, when a large number of genes are investigated, utilization of current in situ hybridization protocol has a technical deficient that the availability is limited.

The above-mentioned PCR method enables detection of nucleic acid with the smallest amount of start materials, and has sensitivity that enables detection on the absence or presence of gene expression for arbitrary gene at a single cell level, however, quantitative comparison on gene products or transcriptional products and such has been difficult according to ordinal methods. In short, including the method described in above-mentioned PCT Publication 2001-509662, some attempts on quantification of gene products or transcriptional products have been reported, all of them are methods for relative quantification, therefore, they are far from absolute quantification method to know how many copies of transcriptional products exist or how many gene products exist. Moreover, in many cases, above-mentioned matter is used for quantification of nucleic acids, and a method has been required for absolute quantification of target substances comprehensively including proteins as well as nucleic acids.

Therefore, the object of this invention is to provide a substrate that enables quantification of target substances as absolute values at cell level, and a method for such quantification.

### MEANS TO SOLVE THE PROBLEMS

To achieve above-mentioned object, the inventors noticed on affinity interaction and made intensive investigation. As the result, the inventors found the substrate and the method for quantification according to this invention.

In other words, the substrate according to this invention is characterized in that it comprises a concentration gradient spotting portion comprising fragments having binding property with probe(s) and having plural concentration gradients, and a specimen setting portion for setting the specimen containing target substance having binding property with said probe.

Moreover, in the preferred embodiment of this invention, said probe is at least one selected from the group consisting of nucleic acid, protein, antibody, antigen and peptide.

Moreover, in the preferred embodiment of this invention, said probe is nucleic acid.

Moreover, in the preferred embodiment of this invention, said concentration gradient spotting portion consists of plural rows.

Moreover, in the preferred embodiment of this invention, among said plural rows, one consists of sense strand and the other consists of antisense strand.

Moreover, in the preferred embodiment of this invention, said substrate comprises glass.

Moreover, in the preferred embodiment of this invention, said specimen is at least one selected from the group consisting of an internal organ, a tissue, an organ, a cell, an organ system and a cell group of interest.

Moreover, in the preferred embodiment of this invention, said target substance is at least one selected from the group consisting of a protein, an antigen, an antibody and a nucleic acid.

Moreover, in the preferred embodiment of this invention, said target substance is a transcriptional product derived from at least one selected from the group consisting of MMP, p53, ki-67, APC, RB, PTEN, INK4a, p14, cadherin, β-Catenin, ras, VEGF and NFκB.

Moreover, the method for quantification of a target substance according to this invention is characterized in that the target substance existing in a specimen containing the target substance having binding property with probe(s) is quantified, based on a standard curve between concentration and intensity obtained by affinity interaction of fragments with said probe, wherein said fragments are those having binding property with said probe and having plural concentration gradients.

Moreover, in the preferred embodiment of this invention, quantification of said target substance is conducted using a laser microscope, a fluorescence microscope or a fluorescence detector.

Moreover, in the preferred embodiment of this invention, said specimen is at least one selected from the group consisting of an internal organ, a tissue, an organ, cell, an organ system and a cell group of interest.

Moreover, in the preferred embodiment of this invention, said target substance is at least one selected from the group consisting of a protein, an antigen, an antibody and a nucleic acid.

Moreover, the other aspect of this invention is characterized in that it comprises the steps of preparing a standard curve between concentration and intensity obtained by affinity interaction of said fragments having plural concentration gradients with said probe, and quantifying the target substance existing in a specimen containing the target substance based on the standard curve.

Moreover, in the preferred embodiment of this invention, affinity interaction of said probe with said fragments having plural concentration gradients and said target substance are conducted as a unit.

### EFFECT OF THIS INVENTION

According to the method of this invention, it is advantageous in that quantification of gene products can be achieved at a single cell level. In cancer tissues, it is known that all cancer cells are heterogenous, and they have diversity including properties such as morphology and metastatic activity. It is assumed that all of such diversity is defined by the diversity in gene expression, therefore, the meaning of identification of transcriptional products at a single cell level deserves high significance.

Moreover, absolute quantification is conducted on individual specimen according to present invention, and so comparison on "numeric value" with past specimen, which had been difficult in conventional relative quantification, becomes possible as an advantageous effect of this invention, . So far, comparison with past data had been difficult, for fluorescent intensity altered owing to difference in condition among each experiment, even in the case of samples from same specimen. According to this invention, numerical identification of the copy number of transcriptional products contained per one cell can be achieved, absolute comparison with past data becomes possible regardless of passage of time.

That is, according to the conventional method for relative quantification, the experimental condition slightly differs among each experiment, therefore, experimental results from plural experiments can not be compared. On the other hand, according to the method for quantification of a target substance according to this invention, standard curve is prepared for each specimen and absolute values can be quantified, thereby the problem of variation on experimental conditions can be resolved.

### BRIEF DESCRIPTION OF THE DARWINGS

Fig.1 shows detection (a photograph) of 28S rRNA with ISH using antisense (a) and sense probes (b) in rat testis. Bars, 1.0 mm (1a) and 0.2 mm (1b).

Fig.2 shows detection of 28S rRNA with ISH using antisense probes in rat seminiferous tubules (a, c). Each image is separated into five tones (b, d) (photograph). Bar, 0.1 mm.

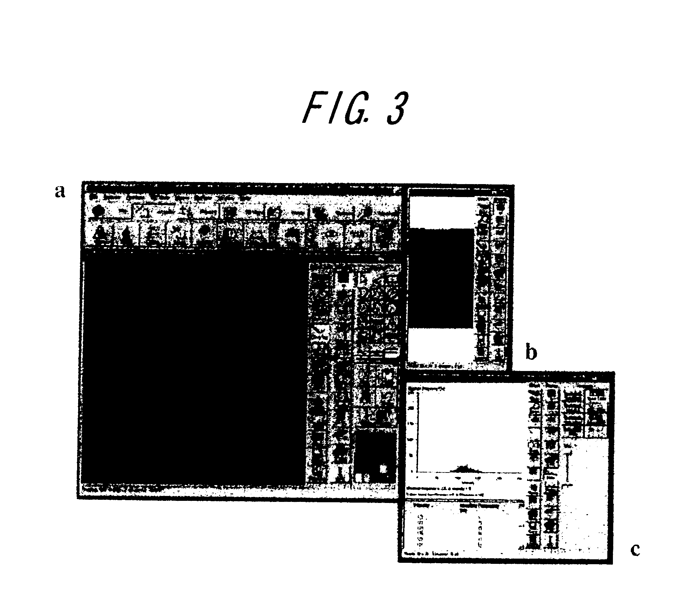

Fig.3 shows quantification of signals. Fig.3a shows the result of ISH of rRNA. Fluorescence is developed by TSA-fluorescein. Fluorescent intensity is measured on one cell encircled by red line. In Fig.3b, the cell encircled by red line is extracted and enlarged, showing that the fluorescent intensity is not uniform in the cell. In histogram of Fig.3c, the horizontal axis represents intensity (0~255) and the vertical axis represents absolute frequency. As shown in the lower table in Fig.3c, frequency is obtained on each intensity, and total intensity in regions encircled by the red line is added. It shows that intensity 123 has frequency of 25 pixel. The conditions for measurement are as follows. Argon laser; wavelength 488nm filter; BP505-550, Optical Slice; 1.8 µm, Pixel depth; 8bit (0-255).

Fig.4 shows detection (photograph) of 28S rRNA with ISH using a tyramide signal amplification system in rat testis. Antisense probe (a), sense probe (b).

Fig.5 shows quantitative analysis of 28S rRNA detected in rat spermatogenic cells. The vertical axis shows the amount of rRNA in the cytoplasm expressed as relative fluorescence intensity, mean pixel intensity. The development progression of a cell is presented horizontally from left to right. P-I indicates pachytene spermatocytes at stage I. Arabic numerals represent steps of spermatids differentiation.

Fig.6 shows one embodiment of the substrate according to this invention.

Fig.7 shows a bar graph of fluorescence intensity, in which oligonucleotide is directly labeled with fluorescein, spotted on slide-glass at various concentrations (1 pmol/µL, 5 pmol/µL, 10 pmol/µL, 50 pmol/µL, 100 pmol/µL), and the fluorescence intensities are measured with the micro-array scanner. It shows that the difference in the fluorescence intensity of the spots can be detected as numeric values on a slide-glass. Moreover, it shows that the fluorescence intensity increases as the concentration of oligonucleotide becomes higher.

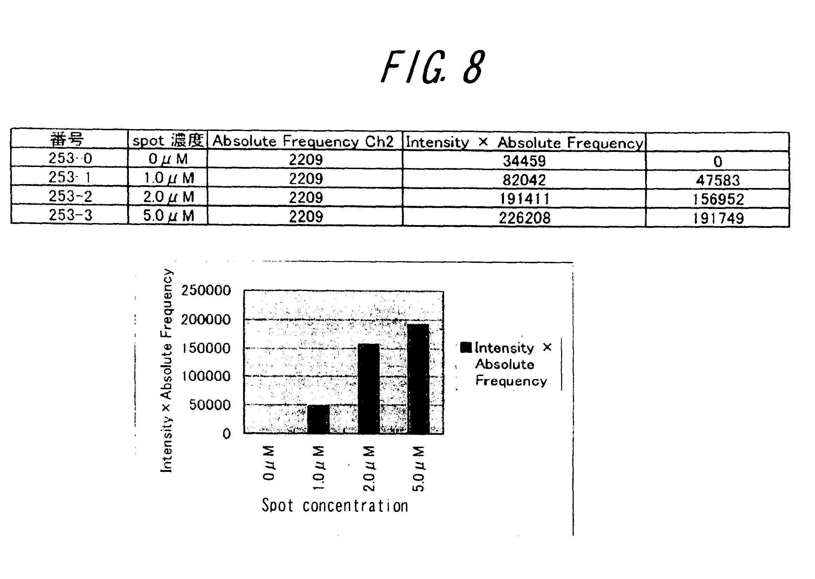

Fig.8 shows relationship between label intensity and concentration obtained by affinity interaction between the fragments having plural concentration gradients and the labeled probe. Absolute Frequency indicates the number of pixel in the measuring range of each spots. Fluorescence intensities of four spots are measured in the range of 2209 pixel of circular shape, all of which have the same dimension. Fluorescence intensities of each pixel (0~255) are measured and all values are added for 2209 pixel, to give the value of Intensity x Absolute Frequency. Fluorescence intensity of 1.0 µM spot is calculated from fluorescence intensity of 1.0 µM by subtracting that of 0 µM, that is, 82042-34459=47583 (the right column in the table). The calculation is conducted for 2.0 µM and 5.0 µM spots in the same manner, which is represented by a bar graph. The calculation is continued for the 1.0 µM spot. The mean value of the 1.0 µM spot (2209 pixel) per 1 pixel can be calculated by 47583/2209=21.5405. As 1.0 µM oligonucleotide is spotted in the range 2209 pixels, calculation can be conducted as 1.0 µM/2209=0.0004526 µM=0.4526nM=453/pM/pixel. Therefore, fluorescence intensity of 21.5405/pixel can be assumed to be corresponding to fluorescence intensity of the target oligonucleotide at the concentration of 453pM/pixel.

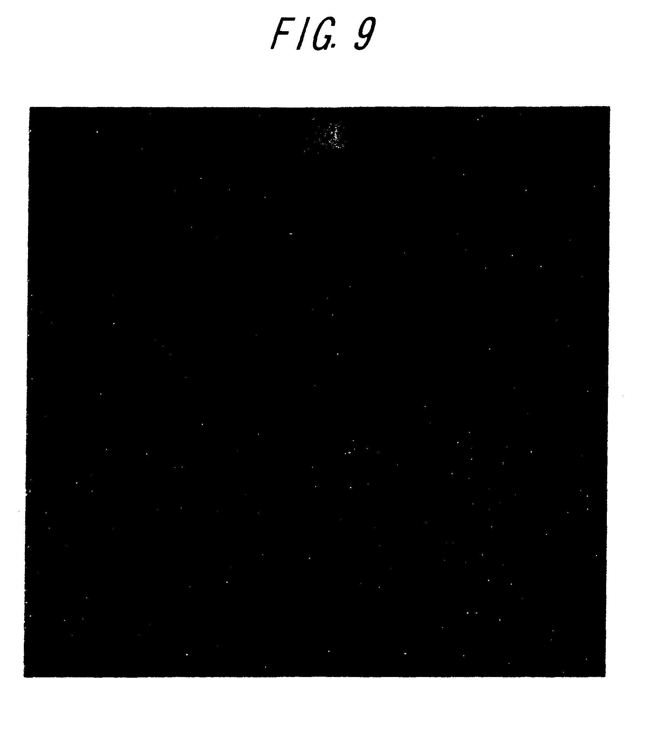

Fig.9 shows fluorescent image (photograph) of the spots.

In Fig. 10A, the range of quantification is selected (the range encircled by red line) for each fluorescent image (photograph), to calculate the fluorescence intensity. Fig. 10A shows the range of analysis for 0 µM of oligonucleotide spot (blank).

Fig. 10B shows the range of analysis for 1 µM of oligonucleotide spot (photograph).

Fig. 10C shows the range of analysis for 2 µM of oligonucleotide spot (photograph).

Fig. 10D shows the range of analysis for 5 µM of oligonucleotide spot (photograph). At the left side of each spot, the antisense oligonucleotide is spotted at the same concentration, as negative control. These spots are not blank, and if the experiment proceeds properly, antisense spots do not exhibit affinity interaction for the antisense labeled probe at all concentrations. A concentration gradient slide was prepared according to the embodiment for the substrate according to Fig.6.

### BEST MODE FOR CARRYING OUT THE INVENTION

The substrate according to present invention comprises a concentration gradient spotting portion comprising fragments having binding property with probe(s) and having plural concentration gradients, and a specimen setting portion for setting the specimen containing target substance having binding property with said probe. The concentration gradient spotting portion comprising fragments having plural concentration gradients mainly functions to quantify the target substance in the specimen. That is, using this concentration gradient spotting portion, by conducting affinity interaction (in the case the probe is nucleic acid, hybridization) of labeled probe (using fluorescent labeling etc.) with the fragment having various concentration gradients, correlation between concentration and intensity can be obtained. Moreover, from the correlation, precise and absolute quantification becomes possible, as described below.

The concentration gradient can be set by an experimenter ad libitum, according to the purpose and the usage of the experiment. In the case the substrate is utilized for quantification of a target substance, it is necessary to set the concentration gradient to include the range of the target substance entirely. If the concentration of the target substance is unknown, it is necessary to prepare sufficient wide range of concentration gradient spot.

In this specification, the wording "probe" is used in the broadest meaning. Usually it means those composed of nucleic acids, for example, it is to be interpreted widely to include antibodies and antigens used in immunoscreening. In short, the probe is not particularly limited, so far as reversible affinity interaction with the fragment or the target substance occurs under certain condition. For example, the examples of such combinations, in other words, as the examples of substances that affinity interacts with the probe, the fragment, or the target substance (substances having binding affinity), enzyme-substrate, coenzyme-enzyme, antigen-antibody, ligand-receptor, DNA-DNA, DNA-RNA, RNA-RNA, PNA-DNA, PNA-RNA etc. and many others can be listed. According to the present invention, these can be chosen ad libitum according to the purpose and the usage of the experiment.

Moreover, in the case a nucleic acid is used as the probe, the present invention will be explained concretely as follows.

For example, as the nucleic acid for the concentration gradient portion, various nucleic acids such as synthetic oligonucleotides, naturally occurring oligonucleotides can be utilized. It is also possible to utilize chemically synthesized RNA strand. In general, in the case of DNA, up to approximately 120 bases can be synthesized, and the length becomes shorter in the case of RNA, probes within such range in length can be utilized. If a longer one is to be spotted, it can be achieved by in vitro transcription of RNA with enzymatic reaction using self-made template, and in the case of DNA, it can be achieved by linearizing or fragmenting plasmids with restriction enzymes after amplified them.

The template utilized at Digoxigenin (DIG) labeling for a probe is often used in the case of chemical synthesis and in the case of excision by an enzyme or in vitro transcription, therefore, the fragment utilized at the spotting portion such as nucleic acid and the labeled probe should be identified, in principle. A probe within such range can be utilized. However, even if they are identical, in the case of RNA and synthetic oligonucleotides, considering difference in sense and antisense orientation, the concept of identical means "complementary", precisely. Moreover, in the case of a double stranded DNA, denaturation is necessary to make single stranded DNA.

The cases where an experimenter may dare to synthesize different sequences and spot them will be as follows. The example of such case may be conducting hybridization under low stringency to search a molecule having homology with probe sequence A, however, such case seems to be rare for in situ hybridization.

Therefore, according to this invention, the fragment to be used for spotting and the (labeled) probe should be identical in principle, or those having complementary sequence, otherwise those having sequence difference at a part can be also applied.

Moreover, the fragment having binding property with probe(s) or the target substance, as described in above-mentioned combination, may be listed: if the probe is an enzyme, the fragment or the target substance is a substrate; if the probe is an antibody, the fragment or the target substance is an antigen protein or an antigen peptide; if the probe is RNA, the fragment or the target substance is RNA or DNA (denatured to single strand). Preferably, said probe is at least one selected from the group consisting of nucleic acid, protein, antigen, antibody, ligand, receptor, enzyme, substrate and coenzyme. If the probe is a nucleic acid, the amount of transcriptional products contained in a tissue section from a specimen can be quantified. That is, if RNA is used as a probe, the amount of its target transcriptional product can be quantified. For example, as the target transcriptional product, transcriptional product derived from at least one selected from the group consisting of MMP, p53 inhibitory oncogene, Ki-67, APC, Rb, PTEN, INK4a, p14, cadherin, β-catenin, ras, VEGF and NFκB can be listed.

Moreover, the transcriptional product is intended to include not only mRNA, but also to include all of rRNA, tRNA, hnRNA and snRNA. Furthermore, it also encompasses those having so-called introns prior to receive processing, which are prior to formation of maturated RNA. Particularly, if rRNA is used as a positive probe, it is preferable in that it can be detected massively in almost all cell species, like such as poly A.

And above, the case of nucleic acid has been explained mainly, the fragment to be used for spotting is not limited to nucleic acids, synthetic proteins and synthetic peptides can be also utilized. In this case, immunohistochemical staining is used instead of in situ hybridization, thereby the amount of antigen protein detected in a tissue can be also quantified.

Moreover, as the target substance, at least one molecule selected from the group consisting of MMP, p53 inhibitory oncogene, Ki-67, APC, Rb, PTEN, INK4a, p14, cadherin, β-catenin, ras, VEGF and NFκB (not only above-mentioned transcriptional products, including proteins etc. derived from these molecules) can be listed.

Here, the specimen is not particularly limited, for example, it may be a section of an internal organ, a tissue, an organ, a cell, an organ system and a cell group to be investigated, it may be also an animal individual or a plant individual. For example, not only relatively small one such as a bacteria or a fungi, but also a whole individual of a mouse or a plant may be also included. As the plant, agricultural products such as a fruit, rice and oats can be also listed. The specimen setting portion is a portion to immobilize said specimen to some extent. Concerning the substrate of this invention, it comprises the gradient spotting portion and the specimen setting portion as described above. When the substrate is used for quantitative method, if the gradient spotting portion and the specimen setting portion are placed on a same surface side (not necessarily a flat surface), the reaction of the fragment and the target substance with the (labeled) probe can be conducted under substantially same reaction condition as described below, thereby more precise and absolute quantification can be achieved.

Moreover, in one embodiment of the substrate according to this invention, said concentration gradient spotting portion consists of plural rows. If the concentration gradient spotting portion consists of plural rows, it enables more precise quantification. For example, it is preferable that among said plural rows, for example, one consists of sense strand and the other consists of antisense strand, in view of confirming normal operation of the experimental system. In this case, as to the antisense-strand probe, fluorescence intensity boundlessly approaches to zero for the spot of antisense strand.

Also the substrate itself is not particularly limited, for example, those having cell adhering effects can be listed, for example, those made from metal (such as made from titanium), those made from glass, those made from plastic and those treated by silane etc. For example, aminosilane forms a coating film by covalent bonding with OH group on the surface of the glass, and positive charge caused by chemically stable amino group is rendered on the glass surface. Owing to the positive charge, strong adherence of tissue section or cell can be achieved. Not only silane treatment, MAS coating, poly-L-lysine coating can be also utilized. When fine figure of a cell specimen is to be observed with fluorescent microscope, glass is preferred as a substrate, in view of adopting a substrate that avoids scattering of fluorescence.

The method for quantification of a target substance according to this invention will be explained thereinafter.

That is, in the method for quantification of a target substance according to this invention, the target substance existing in a specimen containing the target substance having binding property with a probe(s) is quantified, based on a standard curve between concentration and intensity obtained by affinity interaction of fragments with said probe, wherein said fragments are those having binding property with said probe and having plural concentration gradients.

Here, as to the definition and the explanation of the terms used in the method for quantification, such as "fragment having plural concentration gradient", "target substance", "specimen", those explained for the substrate according to this invention as described above can be used for this method for quantification without modification.

Concerning the probe and the fragment having plural concentration gradients, it is premised that they interact each other according to their affinity. As the examples of the (labeled) probes and the fragment having plural concentration gradients, as described above, enzyme-substrate, coenzyme-enzyme, antigen-antibody, ligand-receptor, DNA-DNA, DNA-RNA, RNA-RNA, PNA-DNA, PNA-RNA etc. and many others can be listed.

Labeled probe is intended to mean a probe that is attached with a label, the probe can be labeled using a proper labeling substance, and not particularly limited. As a labeling substance, fluorescent substances such as fluorescein, digoxigenin (DIG), isotope, Cy3 and Cy5 etc. can be listed. To achieve succeeding and final fluorescent development, rhodamine, fluorescein, Cy3, Cy5, Alexa488 and Alexa546 etc. can be utilized.

Moreover, in the method for quantification a target substance according to this invention, quantification of said target substance may preferably be conducted with a laser microscope, a fluorescent microscope or a fluorescent detector. Because such quantification is conducted with a laser microscope, a fluorescent microscope or a fluorescent detector, it is not necessary to use an isotope, thereby the cost can be reduced, and utilization for example in a hospital becomes possible. Moreover, it is advantageous that data including information on two-dimensional location effects, for example the localization of cell to be quantified existing in the tissue, can be obtained by utilizing them at final step. It can be also used for diagnosis of pathological specimen.

Moreover, in the preferred embodiment of the method for quantification of a target substance according to this invention, the specimen is at least one selected from the group consisting of an internal organ, a tissue, an organ, a cell, an organ system and a cell group of interest. As to explanation for the specimen, the explanation on the substrate according to this invention can be referred to. Moreover, in the preferred embodiment, said target substance is at least one selected from the group consisting of a protein, an antigen, an antibody, a peptide, a nucleic acid and a target transcriptional product. As to the target substance, above explanation on the substrate according to this invention can be directly referred to.

In another aspect of the method for quantification of a target substance according to this invention, using the substrate according to this invention described above, a standard curve between concentration and intensity can be prepared by affinity interaction of said fragments having plural concentration gradients with said probe, and said target substance existing in the specimen containing the target substance having binding property with said labeled probe can be quantified base on the standard curve. As to "substrate according to this invention", please refer to the explanation described above. Except that the substrate according to this invention is used, the explanation on the method for quantification of a target substance according to this invention described above can be directly used.

In a preferred embodiment, affinity interaction (in the case of nucleic acid, hybridization) of said labeled probe with said fragments having plural concentration gradients and said target substance can be conducted as a unit. By conducting the affinity interaction as a unit, affinity interaction for the standard curve and affinity interaction of the target substance with the labeled probe can be conducted under substantially identical condition, thereby more precise quantification can be achieved.

Moreover, when the substrate according to this invention is used for the method for quantification of a target substance as described above, for example when nucleic acid is used as a probe, it is very difficult to make identical conditions for hybridization. Therefore, in view of achieving more precise quantification, it is desirable to hybridize the spotting portion and the tissue under identical conditions and to observe by a confocal laser scanning microscope under identical conditions. That is, not only adjustment of the microscope to identical condition is not easy, in addition, laser decreases its brightness day by day, then in strict, making observation at a same day at a same time is desirable, in view of achieving constant condition.

Then one embodiment of the method for quantification of a target substance according to this invention will be explained in the following, by reciting the example of using nucleic acid as a probe.

(1) At first, a portion of a gene sequence (cDNA sequence) of the gene to be investigated (here refereed to gene A), that is, gene sequence of approximately 20 to 50 bases in length (a specific portion not having homology with other genes) may be chemically synthesized, and it can be spotted on a slide glass at various concentrations. It is referred to concentration gradient spotting portion.

(2) Then tissue specimen is attached near the concentration gradient spotting. In situ hybridization is conducted for the tissue specimen and the spotting as a unit. Conventional in situ hybridization is conducted for the tissue specimen. In the conventional in situ hybridization, the presence or absence of expression of the target gene (ordinary, only gene A) that hybridizes with the probe for detection of gene A, in other words, the presence and absence of mRNA transcribed from DNA is detected for individual cells. Here when the amount of mRNA (transcriptional product) is large, the staining will be strong, and when the amount of mRNA is small, the staining will be weak.

(3) As to the last color development process of in situ hybridization that detects the presence or absence of gene expression, it may be changed to fluorescent color development. For example, by measuring the fluorescent intensity by a confocal laser scanning microscope, the intensity may be obtained as numerical value for individual cells, or for the smallest unit (so-called dot) of digital information measurement.

(4) The same operation may be conducted for the spots, and the intensity for individual spots may be obtained. As the concentration of the spot can be estimated previously (i.e. the number of synthesized genes existing in the spot), the number of existing target gene (absolute value) can be determined for individual cells, or for individual dots in the cell.

### EXAMPLE

In the following this invention will be explained in detail by the examples, it is not intended to restrict present invention to the following examples.

### EXAMPLE 1

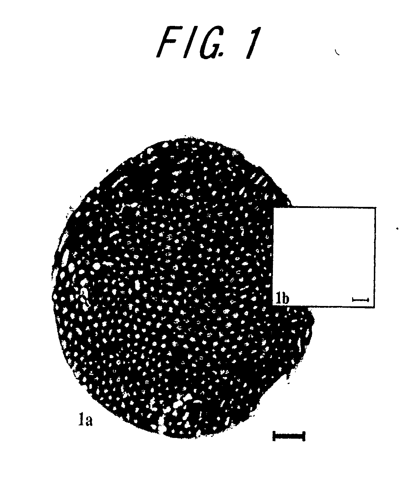

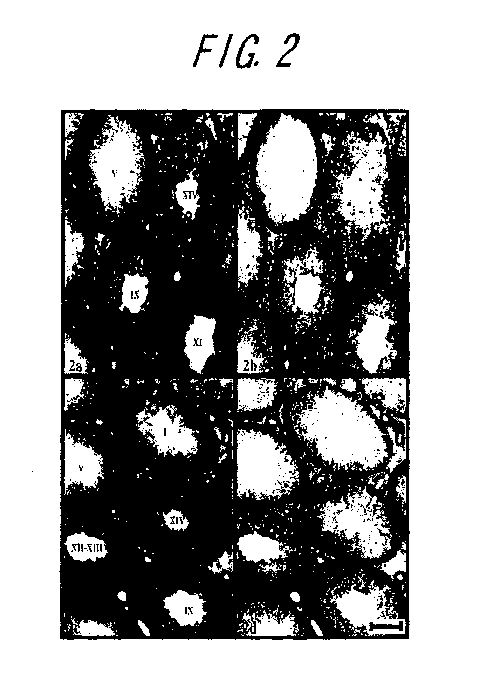

From the knowledge of previous basic research, using the obtained image of the specimen (Fig. 1-1a) which was fixed with Bouin's fixative and hybridized with DIG labeled probe, quantification and analysis of the ISH signal intensity was easily achieved by "posterization" (Fig.2).

The amount of rRNA hybridized with the probe was the highest in early primary spermatocytes, which is succeeded by pachytene spermatocytes, diplotene spermatocytes, late secondary spermatocytes and spermatids. The amount decreased in metaphase, anaphase and telophase of meiosis, reached to the lowest level at early step 1 spermatids, and it slightly increased during spermiogenesis. Staining rRNA by ISH provided an effective parameter for quantitative analysis of mRNA at tissue section and evaluation on the amount of mRNA which can be hybridized.

Moreover, in this experiment, for the quantitative analysis of ISH signal, signal detection was conducted by fluorescent microscope using tyramide signal amplification system (PerkinElmer Japan).

In this model experiment, rRNA (ribosomal RNA) was selected as the target RNA to be hybridized on paraffin section.

### (Material and Methods)

### Tissue preparation

Adult male Wister rats (CLEA Japan : Osaka, Japan) were injected IP with 130 IU/kg heparin and then anesthetized with pentobarbital (50 mg/kg). After fifteen minutes, they were perfused via the left ventricle with Bouin's fixative (375 ml saturated aqueous picric acid, 125 ml 37% w/w stock formaldehyde, and 25ml glacial acetic acid). The testes were carefully isolated and immersed in the same fixative for more than 6hr. The specimens were dehydrated with ethanol, substituted with benzene, and embedded in paraffin. Paraffin sections (5 µm thick) were mounted on silane-coated slides.

### Probe preparation

A well-preserved segment (accession number #V01270; nucleotide number 6035-6068, 5'-gccgccgcaggtgcagatcttggtggtagtagca-3', total 34-mer) of the DNA sequence of rat 28S rRNA was used for the detection of rRNA previously reported by Yoshii et al,(1995) (Yoshii A, Koji T, Ohsawa N, Nakane PK (1995). In situ localization of ribosomal RNAs is reliable reference for hybridizable RNA in tissue sections. Journal of Histochemistry and Cytochemistry 43: 321-327.) An oligonucleotide probe (34-mer) labeled with digoxygenin at the 5' end complementary to the segment of 28S rRNA was obtained from Takara Bio Inc., Japan, by custom synthesis. It is referred to here as the DIG probe.

For the control experiment, unlabeled probes or DIG-labeled 34-mer oligonucleotides of random sequences were prepared.

An alternative DIG-labeled probe was prepared using a multialbeling method (Sasaki et al.1998;Mori et al.2001) as follows. Annealed double stranded 5'-phosphsrylated oligonucleotides containing sense (5'p-aattcgccgccgcaggtgcagatcttggtggtagtagca-a-3') and antisense (5'p-agctt-tgctactaccaccaagatctgcacctgcggcggc-g-3') sequences with EcoR I or Hind III restriction sites as protruding cohensive ends (total 40-mer) were obtained from manufacturer as lyophilized products by custom synthesis. They were ligated to plasmid pBluescript I KS(-), E.coli DH5α strain was transformed by the plasmid, E.coli strain containing the plasmid of purpose was sub-cloned, and the plasmid was isolated and purified. Then subcloned into pBluescript I KS(-). The resulting plasmid DNA was linearized with EcoR I or Hind III and used as a template for the synthesis of labeled sense or antisense riboprobes. DIG-labeled riboprobes were synthesized by in vitro transcription using T3/T7 RNA polymerase in the presence of DIG-UTP according to the instructions provided by the manufacturer of the RNA labeling kit produced by Roche (Roche Diagnostics; Mannheim, Germany). After DNase treatment, the product was ethanol-precipitated and the pellet of DIG labeled probe was resuspended in the hybridization mixture. Limited hydrolysis after Dnase treatment was omitted because the probes were less than 100-mer long. These probes are referred to as ML probes.

A well-preserved segment (accession number #V01270; 6035-6068, 5'-gccgccgcaggtgcagatcttggtggtagtagca-3', total 34-mer) of rat 28S rRNA was used for the detection of rRNA previously reported by Yoshii et al,(1995) (Yoshii A, Koji T, Ohsawa N, Nakane PK (1995). In situ localization of ribosomal RNAs is reliable reference for hybridizable RNA in tissue sections. Journal of Histochemistry and Cytochemistry 43: 321-327.). DIG-labeled riboprobes were synthesized by in vitro transcription using T3/T7 RNA polymerase in the presence of DIG-UTP according to the instructions provided by the manufacturer of the RNA labeling kit used (Roche Diagnostics; Mannheim, Germany). After DNase treatment, the product was ethanol-precipitated and the pellet was resuspended in the hybridization mixture.

### Hybridization

Sections were de-paraffined with toluene and ethanol, then treated sequentially with 0.2 N HCl, 1 µg/ml proteinase K in 2 mM CaCl₂ and Tris-HCl buffer (pH 7.5), and 2 mg/ml of glycine in Dulbecco's PBS and were acetylated with 0.25% acetic anhydride in 0.1 M triethanolamine (pH 8.0) to prevent nonspecific binding of the probes. The slides were washed with 2 X SSC (1 X SSC = 0.15 M NaCl and 0.015 M sodium citrate) and then prehybridized in a solution containing 50% formamide and 2 X SSC at 45°C for more than 1hr. Hybridization was performed on slides using 20 µl of a hybridization mixture containing 50% formamide, 2 X SSC, 1mg/ml tRNA, 0.5 mg/ml sonicated salmon sperm DNA, 2mg/ml bovine serum albumin, 10% dextran sulfate, and the riboprobe (~2 µl/ml) at 45°C for 16hr in a moist chamber. The slides were then rinsed three times with 2 X SSC and 50% formamide at 45°C for 20 min each. After further rinsing of the slides with 1 X SSC, anti-DIG alkaline phosphatase conjugate (1:2500) was placed on the sections at room temperature for 2 hr. After more rinsing of the slides, the enzyme-catalyzed color reaction was continued overnight with 5-bromo-4-chrolo-3-indolyl phosphate and nitroblue tetrazolium salt according to the instructions provided with the kit. The sections were counterstained with methyl green and observed with a light microscope.

For the classification of spermatogenesis in rat testes, the serial sections were stained with periodic acid-Schiff (PAS)-hematoxylin.

After hybridization, slides were treated with RNase solution and TNB blocking buffer. Anti-DIG-POD conjugate (1:1500) was placed on the sections 4°C overnight. After more rinsing of the slides with TNT washing buffer, fluorophore-tyramide (TSA) was added at room temperature for 15 min, rinsed and mounted with anti-fade. For the classification of spermatogenesis in rat testes, the serial sections were stained with periodic acid-Schiff (PAS)-hematoxylin.

### Quantification of Signals

The stained sections were observed under Zeiss confocal Lazer Scanning Microscope (LSM510) using a Plan-Neofluor 40 x 0.75 lens. The signal intensity in the cytoplasm of each spermatogenic cell (primary and secondary spermatocytes and spermatids) was measured by software for the LSM 510. Histogram of the intensity was obtained from the regions of interest in the cytoplasm. The regions were defined by the closed drawing elements (Fig.3a), extracted (Fig.3b), and then converted to tables (Fig.3c) and transferred to an Excel spreadsheet. The signal intensity was calculated and expressed as mean pixel intensity (0-255). The detector gain was sufficiently decreased so that there were no pixels showing maximal intensity.

### Result and Discussion

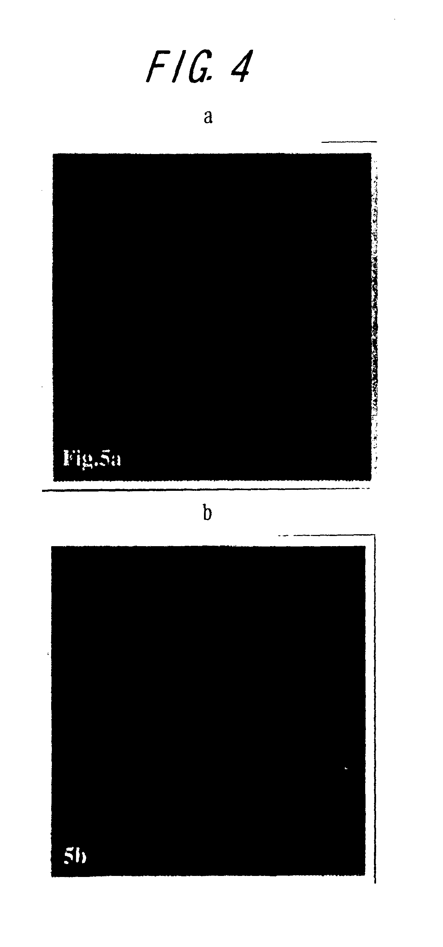

Fig.4a shows detection of rat testis hybridized with antisense probe of rRNA. Fig.4b shows detection of rat testis hybridized with sense probe of rRNA, in which no specific signal was observed.

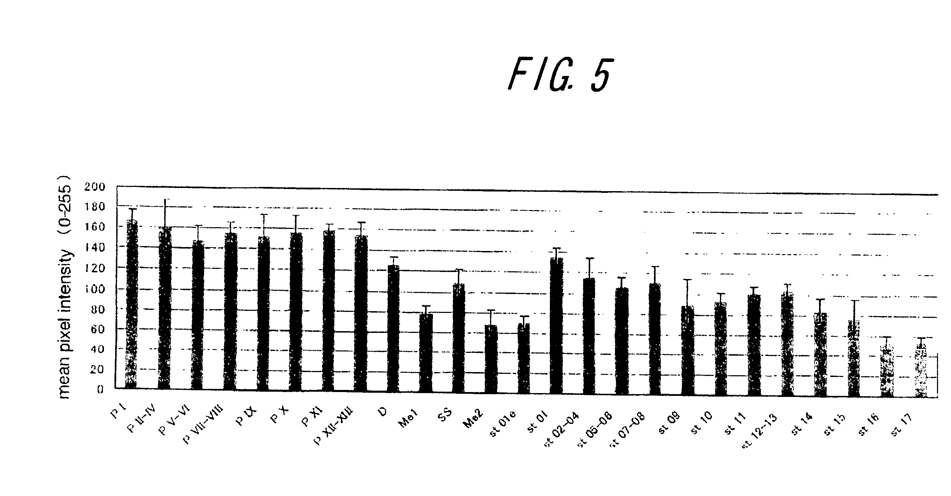

To quantify RNA signals, it was essential that the signals were not distributed unevenly among the seminiferous tubules. It was postulated that spermatogenic cells at the same stage of development in adjacent tubules express the same level of rRNA. We obtained reproducible results in serial sections of the same preparation in the previous study. Analysis of rRNA amounts in spermatogonia and early spermatocytes were not performed because the cell numbers and cell sizes were too small to analyze. The mean pixel intensity value of each cell cytoplasm was obtained and the results are summarized in Fig.5. The signal intensity in each spermatogenic cell cytoplasm, expressed as the mean pixel intensity, was 147-166 in primary spermatocytes, 125 in diplotene spermatocytes, 108 in secondary spermatocytes and 53-135 in speramtids. The intensity in metaphase, anaphase and telophase of meiotic divisions and early step 1 speramtids was 68-70. The amount of rRNA in these spermatogenic cells presented in this study was consistent with the previous data. Many problems had been overcome for the quantification of mRNA in tissue sections. The first problem was whether or not there was linearity between the probe concentration and the ISH signals. The next problem was whether or not the number of probe molecules per unit area or per cell cross-section accurately reflected the cellular mRNA content. And the other problem was that exact measurement of changes in cell volume, the volume of the structure, or the number of labeled cells was necessary for the assessment of the exact change in the gene expression of given tissue. The present model experiments using fluorescent detection system may bring a new insight to solve these problems underlying the quantification of various signals in tissue sections.

### EXAMPLE 2

From the results of EXAMPLE 1, it was suggested that there may be some relationship between the probe concentration and the signal intensity. From such point of view, investigation was performed again.

At first, the substrate according to present invention was prepared. A slide-glass was used as the substrate.

In the same manner as preparation of conventional micro-array slide-glass, a series of diluted DNA, RNA and oligonucleotide prepared for the gene to be investigated with various concentrations were spotted on a slide-glass using a spotter. As spotting is conducted by making spots of circular shape of a same dimension (diameter of 220 µm) within error of 1 nL, the copy numbers of nucleic acid contained in the spot can be calculated.

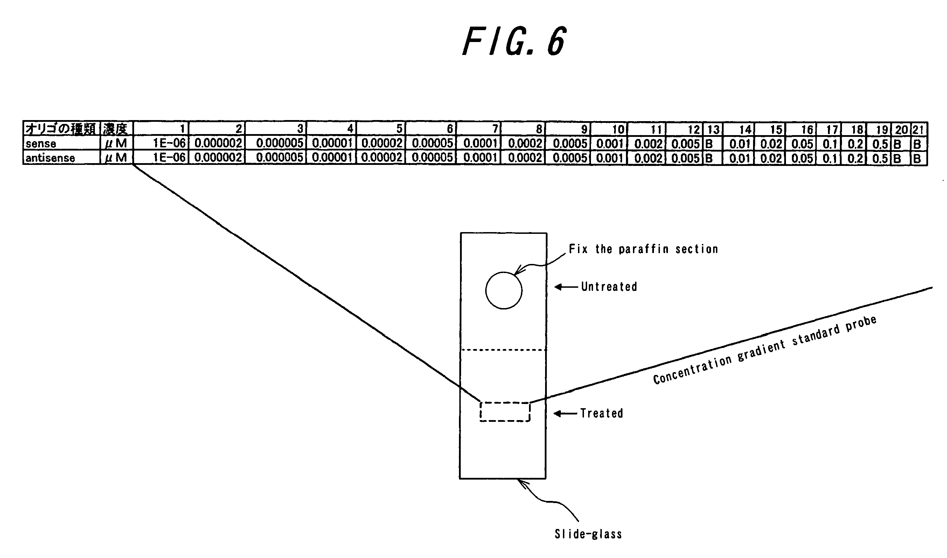

Next, one surface of the slide-glass (the same slide-glass on which concentration gradient spot was made) was silane coated according to conventional method, and paraffin section of an internal organ, a tissue, or an organ of interest or a frozen section was attached onto the slide-glass. Plural rows consisting of sense and antisense strands were adopted for the concentration gradient spotting portion. The result is shown in Fig.6. Fig.6 shows one embodiment of the substrate according to this invention.

In situ hybridization was carried out for the spot of dilution series and the specimen as a unit. A cRNA probe labeled with DIG was prepared for the gene to be investigated, and fluorescent staining was conducted using anti-DIG antibody conjugated with peroxidase, TSA-fluorescein, TSA-Alexa etc.

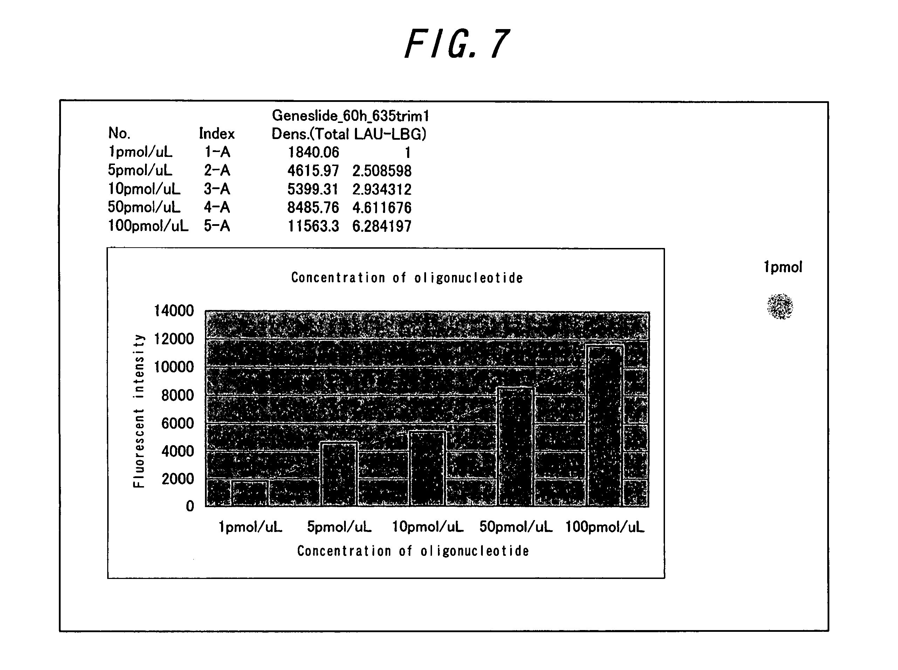

The fluorescent intensities on the section and the spot of dilution series were measured by a confocal laser scanning microscope (Carl Zeiss, LSM510 etc). The fluorescent intensities of the spot of dilution series and the cell of interest on the tissue section were compared, thereby the copy numbers of the transcriptional products contained in the respective cells can be determined. Fig.7 shows the relationship between label intensity and oligonucleotide concentrations of the labeled fragments, wherein the fragments have plural concentration gradients. Using such standard curve, the fluorescent intensity of a cell on said section can be compared and investigated.

### EXAMPLE 3

Next, a standard curve was obtained from affinity interaction between fragments having plural concentration gradients and labeled probe, thereby quantification of a target substance was actually conducted. Fig.8 shows the standard curve obtained from affinity interaction between fragments having plural concentration gradients and labeled probe.

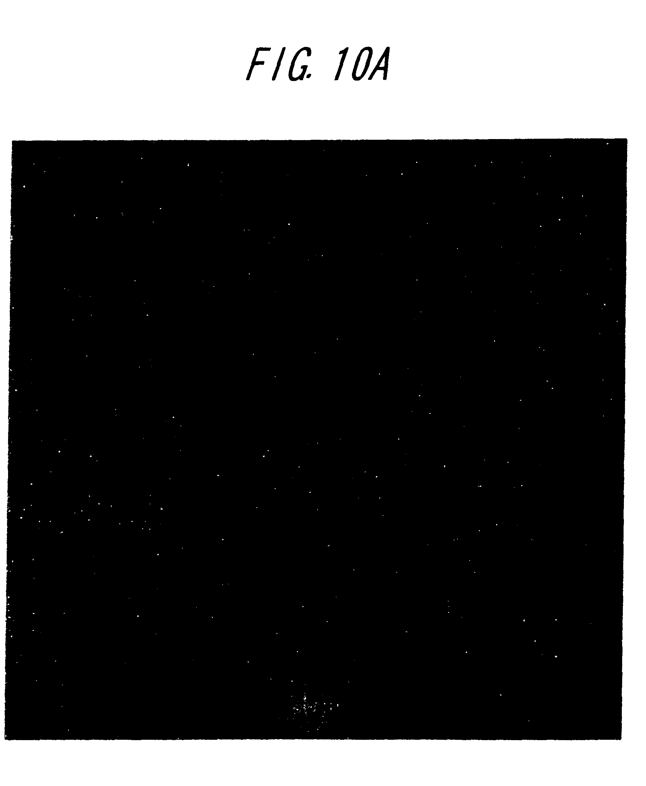

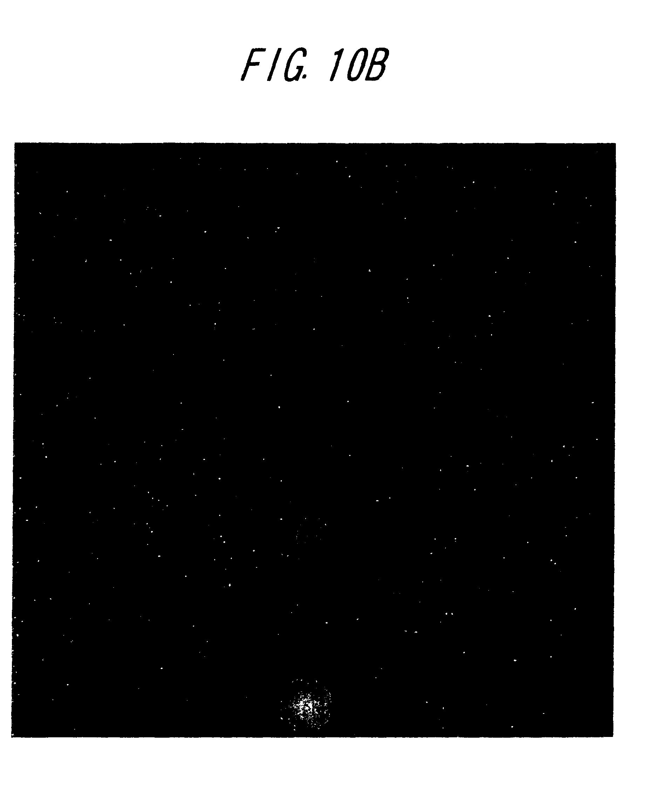

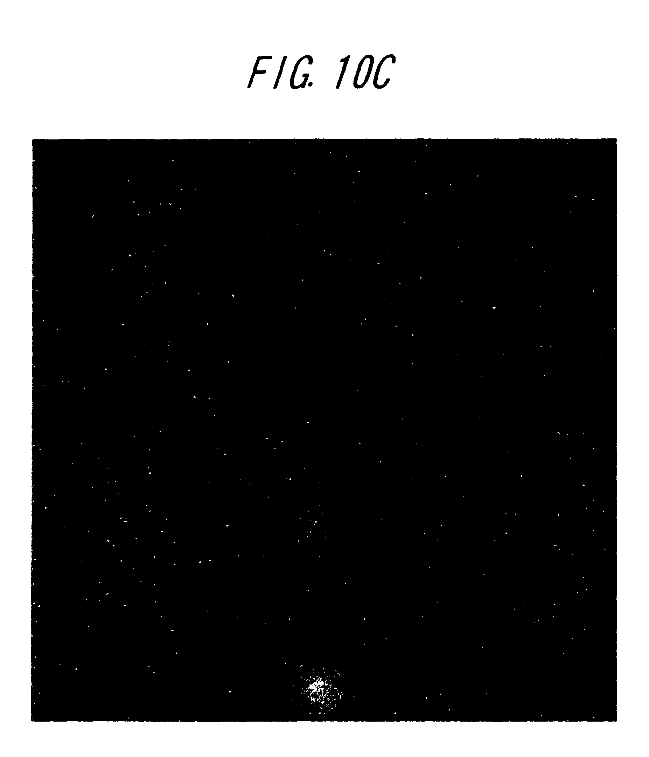

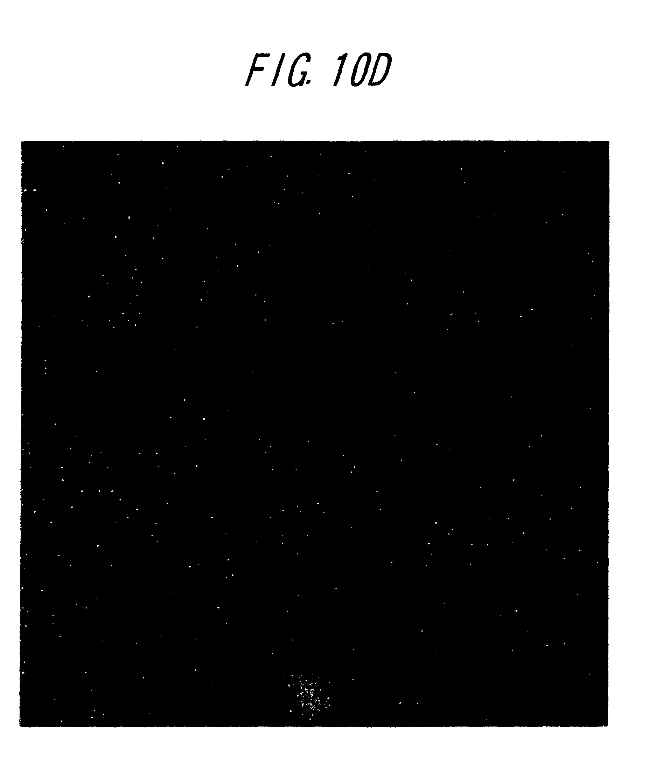

In Fig. 10, the region for quantification was selected (the range enclosed by the red line) for each fluorescent image of the spot, for the purpose to calculate the fluorescent intensity. Fig. 10 (1) shows the range for analysis of 0 µM oligonucleotide spot (i.e. blank). Fig. 10 (2) shows the range for analysis of 1 µM, Fig. 10 (3) shows the range for analysis of 2 µM, Fig. 10 (4) shows the range for analysis of 5 µM of oligonucleotide spot, respectively. The corresponding concentrations of antisense oligonucleotides are spotted at the left side of each spots, as negative controls. These spots are not blank, and if the experiment proceeds properly, antisense spots do not exhibit affinity interaction to the antisense probe at all concentrations. A concentration gradient slide was prepared according to the embodiment for the substrate according to Fig.6. As the result, following matters became possible.

That is, for the quantification of individual transcriptional product of the specimen such as cancer cells, section of cancer tissues obtained by operation or biopsy can be prepared, and the amount of expression of MMP, cadherin, p53 suppressive oncogene, which reflect the malignancy of cancer cell, can be quantified as absolute values at cell level. The result can be used for assumption of prognosis and for determination of therapeutic measures as a part of tailor-made medical procedures.

Especially, for the reason that this quantification can be conducted on a tissue section in addition to conventional pathological diagnosis, it is advantageous that transcriptional products of arbitrary genes can be quantified, thereby pathological tissue malignancy can be correlated with the copy numbers of the gene products.

In other words, diagnosis of malignancy of cancer, which has been entrusted to specialists on pathology, can be presented by numerical values by quantification of gene product. That is, the malignancy can be diagnosed without training of clinical pathology.

### INDUSTRIAL APPLICABILITY

Pathological examination is a requisite technique even now for diagnosis of many intractable diseases, not only examination of cancer but also autoimmune diseases etc. Despite of it, the number of pathologist is small and a great amount of times and labor are needed for pathological diagnosis. If the method according to this invention are used widely, in addition to tissue diagnosis by pathologists, the expression of genes involved in pathological status can be expressed as "numerical values" at cell level, it will deserve social significance.

Moreover, as to quality verification of agricultural and marine products, quality verification by quantification of gene products by conventional methods has been difficult. Because the sizes of the individuals and organs are different in cultured fishes or livestock depending to difference in age or given food, even in same species. However, even if the sizes of individuals and organs are different, the sizes of individual cells are assumed to be identical in same species. Therefore, according to this invention, tissue section can be prepared and absolute values of gene products can be quantified for arbitrary gene at the level of a single cell, as the advantageous effect of this invention.

## Claims

1. A substrate comprising a concentration gradient spotting portion comprising fragments having binding property with probe(s) and having plural concentration gradients, and a specimen setting portion for setting the specimen containing target substance having binding property with said probe.

2. The substrate according to Claim 1, wherein said probe is at least one selected from the group consisting of nucleic acid, protein, antibody, antigen and peptide.

3. The substrate according to Claim 1 or Claim 2, wherein said probe is nucleic acid.

4. The substrate according to Claim 1, wherein said concentration gradient spotting portion consists of plural rows.

5. The substrate according to Claim 3 or Claim 4, wherein among said plural rows, one consists of sense strand and the other consists of antisense strand.

6. The substrate according to any one of Claim 1 to Claim 5, wherein said substrate comprises glass.

7. The substrate according to any one of Claim 1 to Claim 6, wherein said specimen is at least one selected from the group consisting of an internal organ, a tissue, an organ, a cell, an organ system and a cell group of interest.

8. The substrate according to any one of Claim 1 to Claim 7, wherein said target substance is at least one selected from the group consisting of a protein, an antigen, an antibody and a nucleic acid.

9. The substrate according to Claim 8, wherein said target substance is at least one selected from the group consisting of MMP, p53, cadherin, β-catenin, ras, VEGF and NFκB.

10. A method for quantification of a target substance, **characterized in that** said target substance existing in a specimen containing the target substance having binding property with probe(s) is quantified, based on a standard curve between concentration and intensity obtained by affinity interaction of fragments with said probe, wherein said fragments are those having binding property with said probe and having plural concentration gradients.

11. The method according to Claim 10, wherein quantification of said target substance is conducted using a laser microscope, a fluorescence microscope or a fluorescence detector.

12. The method according to Claim 10 or Claim 11, wherein said specimen is at least one selected from the group consisting of an internal organ, a tissue, an organ, a cell, an organ system and a cell group of interest.

13. The method according to any one of Claim 1 to Claim 7, wherein said target substance is at least one selected from the group consisting of a protein, an antigen, an antibody and a nucleic acid.

14. A method for quantification of said target substance using the substrate according to any one of Claim 1 to Claim 9 **characterized in that** it comprises the steps of preparing a standard curve between concentration and intensity obtained by affinity interaction of said fragments having plural concentration gradients with said probe, and quantifying the target substance existing in a specimen containing the target substance based on the standard curve.

15. The method according to Claim 15 wherein affinity interaction of said probe with said fragments having plural concentration gradients and said target substance are conducted as a unit.

## Amended claims

### Amended claims under Art. 19.1 PCT

**15.** Amended) The method according to Claim 14 wherein affinity interaction of said probe with said fragments having plural concentration gradients and said target substance are conducted as a unit.
